# EUROPÄISCHE PATENTANMELDUNG

(11) **EP 4 209 188 A1**
(43) Veröffentlichungstag der Anmeldung: **12.07.2023**
(21) Anmeldenummer: 22150600.9
(22) Anmeldetag: 07.01.2022
(51) Int. Cl.: A61B 18/04, A61B 18/14

(54) **PLASMASONDE**

(71) Anmelder: Erbe Elektromedizin GmbH, 72072 Tübingen (DE)
(72) Erfinder: HEYM, Johannes, 72070 Tuebingen (DE)
(74) Vertreter: Rüger Abel Patentanwälte PartGmbB

(57) **Zusammenfassung**

Die Erfindung schafft ein Konzept zur zuverlässigen und einfachen Verbindung eines hitzefesten Endstücks (17) an einem Schlauchkörper (18), insbesondere einem mehrlumigen Schlauchkörper (18). Die erfindungsgemäße Verbindungstechnik ist einfach und verlässlich und führt zu langlebigen Sonden hoher Qualität.

## Beschreibung

Die Erfindung betrifft eine Plasmasonde, insbesondere zur endoskopischen Behandlung menschlicher oder tierischer Patienten, sowie ein Verfahren zur Herstellung einer solchen Plasmasonde.

Aus der DE 100 30 111 B4 ist eine gattungsgemäße Plasmasonde bekannt, die einen flexiblen, ein einziges Lumen umschließenden Schlauch aufweist, der an seinem distalen Ende mit einem aus Keramik bestehenden Mundstück versehen ist. In dem Schlauch ist ein elektrischer Leiter angeordnet, der an seinem distalen Ende eine zentral in dem Mundstück stehende Elektrode bildet. In Betrieb strömt ein geeignetes Gas in distaler Richtung durch das Lumen und wird an der Elektrode ionisiert, sodass ein Plasmastrahl das Mundstück verlässt.

Zur Befestigung des Mundstücks an dem Schlauch weist dieses einen Rohrschaft auf, der sich in den Schlauch hinein erstreckt.

Die DE 696 32 080 T2 offenbart ebenfalls eine Plasmasonde mit einem Schlauchkörper, der an seinem distalen Ende mit einem aus Keramik oder PTFE bestehenden Endstück versehen ist. Wiederum weist das Endstück einen Schaft auf, der sich in den Schlauch hinein erstreckt, um eine Verbindung zwischen dem Endstück und dem Schlauch herzustellen.

Zur Zentrierung der Elektrode sind im Stand der Technik unterschiedliche Maßnahmen vorgesehen. Gemäß DE 696 32 080 T2 kann die Elektrode einen schraubenförmig gewundenen Abschnitt aufweisen, dessen Windungen innen an dem Schlauch oder an dem Endstück anliegen und deren letzte Windung eine stiftförmige Elektrode zentral dem Endstück hält.

Gemäß DE 100 30 111 B4 kann zur Zentrierung der Elektrode alternativ ein plättchenförmiges Element vorgesehen sein, dessen laterale Kanten sich innen an dem Schlauch abstützen und dessen distale Spitze selbst als Elektrode dient.

Beide Konzepte stoßen mit zunehmender Miniaturisierung der Sonden an Grenzen.

Davon ausgehend ist es Aufgabe der Erfindung, ein verbessertes Konzept zur Gestaltung des distalen Endes einer Sonde anzugeben.

Diese Aufgabe wird mit der Sonde mit den Merkmalen des Anspruchs 1 gelöst. Anspruch 15 betrifft ein besonders geeignetes Verfahren zur Herstellung einer Sonde nach Anspruch 1.

Die erfindungsgemäße Plasmasonde weist einen Schlauchkörper auf, der mindestens ein Lumen umschließt. Radial nach außen ist dieses Lumen durch einen Mantelbereich des Schlauchkörpers zur Umgebung hin abgegrenzt. Der Begriff "radial" bezieht sich dabei auf die Radialrichtung, wenn die Längserstreckung der Plasmasonde als Axialrichtung verstanden wird. Der Mantelbereich ist über mindestens einen Wandbereich mit einem Mittelbereich verbunden, der zentral oder auch leicht außerhalb der Mitte des Schlauchkörpers angeordnet sein kann. Vorzugsweise ist nicht lediglich ein Wandbereich, sondern sind mehrere Wandbereiche, beispielsweise drei, vorgesehen, die den Innenraum des Schlauchkörpers in mehrere Lumina unterteilen und zugleich den Mittelbereich des Schlauchkörpers in gewünschter, zum Beispiel zentraler, Position halten. Der Mittelbereich, die Wandbereiche und der Mantelbereich sind vorzugsweise Bestandteil des einstückigen Schlauchkörpers, d.h. sie bestehen aus dem gleichen Material und schließen naht- und fugenlos aneinander an.

An dem distalen Ende des Schlauchkörpers ist ein Endstück angeordnet, das insbesondere aus einem hitzefesten Material, wie beispielsweise einem hitzefesten Kunststoff oder Keramik ausgebildet sein kann. Das Endstück weist eine zentrale Durchgangsöffnung mit einer Mündung auf, die einen Gas- bzw. Plasmaaustritt gestattet. Die Mündung kann an der distalen stirnseitigen Endfläche des Endstücks oder auch in einem seitlichen Bereich desselben vorgesehen sein. Es können auch mehrere Mündungen vorgesehen sein.

Das Endstück weist einen Rohrschaft auf, der sich in den Schlauchkörper hinein erstreckt. Dazu ist zwischen dem Mantelbereich des Schlauchkörpers und dem einen oder den mehreren Wandbereichen ein Schlitz ausgebildet, der sich von dem distalen Ende des Schlauchkörpers in proximaler Richtung um eine Länge in den Schlauchkörper hinein erstreckt, die vorzugsweise mindestens so groß ist wie die in Axialrichtung zu messende Länge des Rohrschafts des Endstücks. Der Rohrschaft ist in diesen Schlitz eingeschoben, wobei er die Wandbereiche radial nach innen verdrängt, sodass diese an der inneren Wandung der Durchgangsöffnung des Rohrschafts anliegen.

Die Wandbereiche des Schlauchkörpers können radial oder auch schräg zur Radialrichtung angeordnet sein. Sind sie schräg angeordnet, können sie den Schlitz für den Rohrschaft leicht freigeben, indem sie sich etwas verbiegen. Sind sie radial angeordnet, geben sie den Schlitz für den Rohrschaft frei, indem sie sich verbiegen und/oder stauchen.

Durch die Anordnung des Rohrschaftes in dem Schlitz zwischen den Wandbereichen und dem Mantelbereich ist die Montage des Endstücks an einem Schlauch mit einem Wandbereich zwischen dem Mantelbereich und dem Mittelbereich besonders einfach. Es muss kein Material aus dem Inneren des Schlauchkörpers entfernt werden. Es genügt entlang der Innenfläche des Mantelbereichs einen Schnitt in dem Wandbereich anzubringen, d.h. den Wandbereich von dem Mantelbereich zum Beispiel durch einen Schnitt zu trennen. Durch die Verengung des freien Lumens des Schlauchkörpers zum einen durch im Rohrschaft des Endstücks und zum anderen durch die gestauchten oder nach innen gebogenen Wandbereiche entsteht in dem Rohrschaft des Endstücks ein Düseneffekt, der für den Betrieb als Plasmasonde vorteilhaft sein kann. Insbesondere wird zu großer Hitzeeintrag in die Plasmasonde wirksam vermindert.

Der Schlauchkörper kann aus einem flexiblen, insbesondere biegeelastischen Kunststoff ausgebildet sein, wie beispielsweise PTFE, PE oder dergleichen. Dabei sind vorzugsweise der Mantelbereich, der Wandbereich (oder die Wandbereiche) und der Mittelbereich gemeinsam urgeformt. Der Schlauchkörper kann zum Beispiel im Ganzen durch Extrusion hergestellt sein.

Durch die vorzugsweise Unterteilung seines Innenraums in zwei, drei oder mehreren Lumina durch die Wandbereiche wird einerseits eine hohe Flexibilität und andererseits erreicht, dass der Gasdurchgang durch den Schlauchkörper bei Biegung desselben auch bei geringen Biegeradien nicht versperrt wird. Dies gilt insbesondere, wenn die Wandbereiche schräg zur jeweiligen Radialrichtung angeordnet sind.

Vorzugsweise ist der Schlauchkörper über seine gesamte, sich von dem proximalen bis zu dem distalen Ende erstreckende Länge mit einem konstanten Querschnitt versehen. Das distale Ende ist das dem Patienten zugewandte zur Behandlung dienende Ende. Das proximale Ende ist das an das speisende Geräte anzuschließende Ende. Der Querschnitt des Schlauchkörpers ist dabei auf eine Fläche bezogen, deren Flächennormale parallel zu der Längsrichtung des Schlauchkörpers orientiert ist. Die Längsrichtung geht von proximal nach distal.

Das Endstück kann wie schon erläutert aus einem hitzefesten Kunststoff oder auch aus Keramik ausgebildet sein. Dies gilt insbesondere bei der Ausbildung der Sonde als monopolare Plasmasonde, bei der eine einzige Elektrode in oder an dem Mittelbereich des Schlauchkörpers gehalten ist und zur Plasmaerzeugung dient. Es ist jedoch auch möglich, das Endstück aus einem Metall auszubilden und zum Beispiel mit einem Neutralleiter zu verbinden. Die Sonde kann dann als Bipolarsonde ausgebildet sein, bei der eine elektrische Entladung zwischen einer zentralen Elektrode und dem Endstück stattfindet. Durch die Entladung strömendes Gas kann dabei ionisiert werden, so dass wiederum ein Plasmastrahl entsteht, der die Sonde am distalen Ende verlässt.

Der Rohrschaft des Endstücks dient der Befestigung des Endstücks an dem Schlauchkörper. Dazu sitzt der Mantelbereich auf der Außenfläche des Rohrschafts während die Wandbereiche innen an der Wandung der Durchgangsöffnung desselben anliegen. Der Mantelbereich kann dabei unter Vorspannung an dem Rohrschaft anliegen, wenn der Außendurchmesser des Rohrschafts etwas größer ist als der Innendurchmesser des Mantelbereichs. Zusätzlich kann an dem Rohrschaft außen eine Verankerungsstruktur vorgesehen sein, beispielsweise in Gestalt von Rippen, Zähnen oder einem Gewinde.

Der Mittelbereich des Schlauchkörpers kann einen elektrischen Leiter enthalten, der sich vorzugsweise durch die gesamte Länge des Schlauchkörpers erstreckt. Der Mittelbereich kann den Leiter umschließend ausgebildet sein. An dem proximalen Ende des Schlauchkörpers kann der Leiter über geeignete Anschlussmittel, zum Beispiel einen Stecker, mit einem elektrischen Generator verbunden sein. An dem distalen Ende kann der elektrische Leiter eine Elektrode bilden oder mit einer Elektrode verbunden sein. Die Elektrode kann beispielswiese als Röhrchenelektrode ausgebildet sein und von dem Leiter getragen und kontaktiert sein. Es ist möglich, die röhrchenförmige Elektrode in den Mittelbereich des Schlauchkörpers zwischen den Leiter und das dadurch nach außen aufgeweitete Kunststoffmaterial des Schlauchkörpers einzuschieben. Die Elektrode ist dadurch unter Verformung des Schlauchs, insbesondere seines Mittelbereichs, in demselben durch Klemmwirkung gehalten. Der Mittelbereich kann dadurch etwas aufgeweitet sein, wodurch der Strömungsquerschnitt in dem Rohrschaft verengt und dadurch die Strömungsgeschwindigkeit des Gases erhöht wird.

Wenngleich nicht zwingend, so ist es doch zweckmäßig, den Leiter vor Anbringung der Elektrode freizulegen, so dass der Leiter zunächst in distaler Richtung aus dem Schlauchkörper ragt. Die Röhrchenelektrode kann auf diesen Leiter zunächst aufgeschoben und dann in den Mittelbereich eingeschoben werden. Bei Verwendung einer Stiftelektrode kann darauf aber verzichtet werden. Die Stiftelektrode wird dann einfach in Nachbarschaft des Leiters in den Mittelbereich eingestochen.

Es hat sich als vorteilhaft herausgestellt, die Elektrode aus einem hitzebeständigen elektrisch leitfähigen Material wie beispielsweise Edelstahl, Hartmetall, unedlem Stahl, Wolfram, Cermet oder elektrisch leitfähiger Keramik auszubilden. Die Elektrode kann zusätzlich mit einer metallischen Beschichtung versehen sein, insbesondere aus einem Metall, dessen Schmelztemperatur niedriger ist, als die Schmelztemperatur des Elektrodenmaterials, wie beispielsweise Silber. Es hat sich gezeigt, dass sich dadurch die elektrische von der Elektrode ausgehende Entladung stabilisieren und deren Entladungsfußpunkt insbesondere auf das distale Ende der Elektrode konzentrieren lässt.

Anspruch 15 ist auf ein besonders einfaches und sicheres Verfahren zur Herstellung der genannten Plasmasonde gerichtet. Dazu wird zunächst der ein- oder mehrlumige Schlauchkörper bereitgestellt. An die Innenseite des Mantelbereichs angrenzend wird die Verbindung zwischen dem Wandbereich und dem Mantelbereich durch einen Schnitt durchtrennt. Der entsprechende Schnitt weist in Axialrichtung eine Länge von mehreren Millimetern auf und ist vorzugsweise in Axialrichtung gemessen länger als der Schaft des Endstücks. Vorzugsweise wird dieser Schnitt ohne Materialabtrag durch ein axial und/oder in Umfangsrichtung bewegtes Messer ausgeführt, sodass weder Späne noch sonstige Partikel (Kunststoffpartikel) entstehen, die in das Lumen gelangen oder an Teilen der Sonde haften und später zu Funktionsstörungen führen könnten.

Nach Erzeugung des Schnitts wird der Rohrschaft des Endstücks in den Schlitz (die Schlitze) eingeschoben, wobei der Wandbereich (oder die Wandbereiche) aus dem Schlitz verdrängt werden. Sie tragen zur Verengung des Strömungsquerschnitts im Bereich des Rohrschafts des Endstücks und somit zur Erhöhung der Strömungsgeschwindigkeit an dieser Stelle bei. Dies fördert die Kühlung des Mittelbereichs der zur Fixierung der Elektrode herangezogen werden kann.

Weitere Einzelheiten vorteilhafter Ausführungsformen der Erfindung ergeben sich aus Unteransprüchen und/oder der den Figuren der Zeichnung nebst zugehöriger Beschreibung. In der Zeichnung zeigen:

Figur 1 ein Gerät mit angeschlossener Sonde, in Prinzipdarstellung,

Figur 2 das distale Ende der Sonde nach Figur 1, in perspektivischer vergrößerter Darstellung,

Figur 3 das distale Ende der Sonde gemäß Figur 2, in Explosionsdarstellung ohne Elektrode,

Figur 4 bis 6 verschiedene Stadien bei der Bereitstellung des Schlauchkörpers der Sonde vor Montage des Endstücks,

Figur 7 den Schlauchkörper in verformten Zustand bei eingeschobenem Endstück, in perspektivischer Darstellung,

Figur 8 den Schlauchkörper nach Figur 7 mit montierter Elektrode, in perspektivischer Darstellung,

Figur 9 das distale Ende der Sonde in längsgeschnittener Darstellung,

Figur 10 die Sonde nach Figur 9 mit schräg zur Radialen angestellten Wandbereichen in Sicht auf das distale Ende der Sonde und

Figur 11 die Sonde in einer Darstellung gemäß Figur 10 jedoch mit radial angeordneten, gestauchten Wandbereichen.

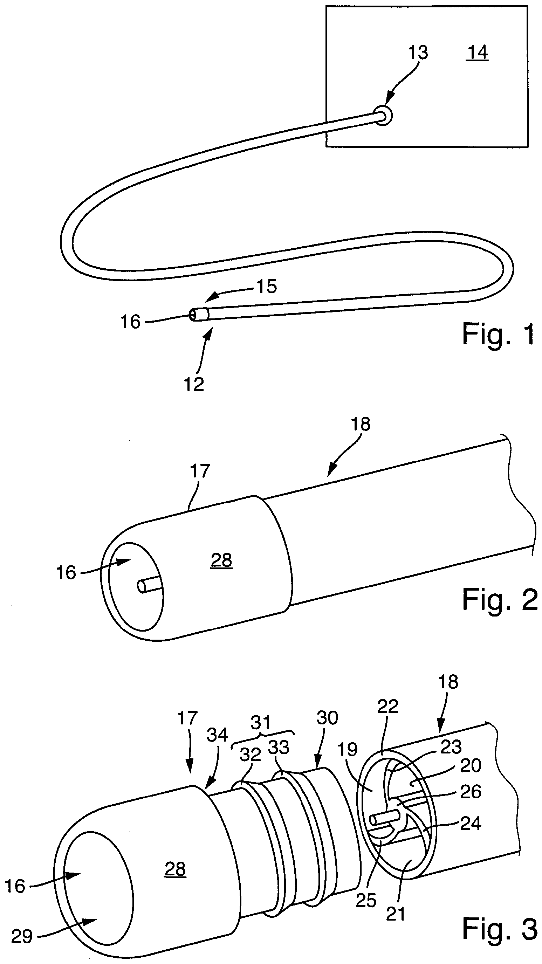

In Figur 1 ist eine Plasmasonde 12 veranschaulicht, deren proximales Ende 13 an ein Gerät 14 zur Speisung der Sonde 12 angeschlossen ist. Die Sonde kann als Plasmasonde, insbesondere Argon-Plasma-Sonde, ausgebildet sein. Das Gerät 14 beinhaltet dann die nötigen Komponenten zur Speisung der Sonde 12, beispielsweise mit hochfrequenter Spannung und Strom und mit einem Gas, insbesondere Inertgas, insbesondere Argon. Anstelle eines einzelnen Geräts 14 können auch mehrere Geräte vorgesehen sein, von denen jedes eine eigene Aufgabe übernimmt, beispielsweise die Speisung der Sonde 12 mit Strom oder die Speisung der Sonde 12 mit einem geeigneten Gas.

Die Sonde 12 weist ein distales Ende 15 auf, das einen Plasmastrahl zur Einwirkung auf biologisches Gewebe eines tierischen oder menschlichen Patienten auslässt. Die Sonde 12 kann dazu beispielswiese durch den Arbeitskanal eines Endoskops oder durch ein geeignetes Körperlumen so positioniert werden, dass das distale Ende 15 der Sonde 12 unmittelbar bei dem zu behandelnden Gewebebereich steht. Der Plasmastrahl der Sonde 12 tritt aus einer distalen Öffnung 16 aus, die aus Figur 2 ersichtlich ist. Die Öffnung 16 ist an einem aus hitzefesten Material bestehenden Endstück 17 ausgebildet, das an dem Schlauchkörper 18 der Sonde 12 gehalten ist. Während der Schlauchkörper 18 aus einem flexiblen Kunststoff wie PTFE, PA12, PEBAX, Polyethylen oder dergleichen besteht, ist das Endstück 17 aus einem typischerweise steifen, d.h. weniger flexiblen, dafür aber hitzefesten Material wie Keramik, hitzefestem Kunststoff oder dergleichen ausgebildet.

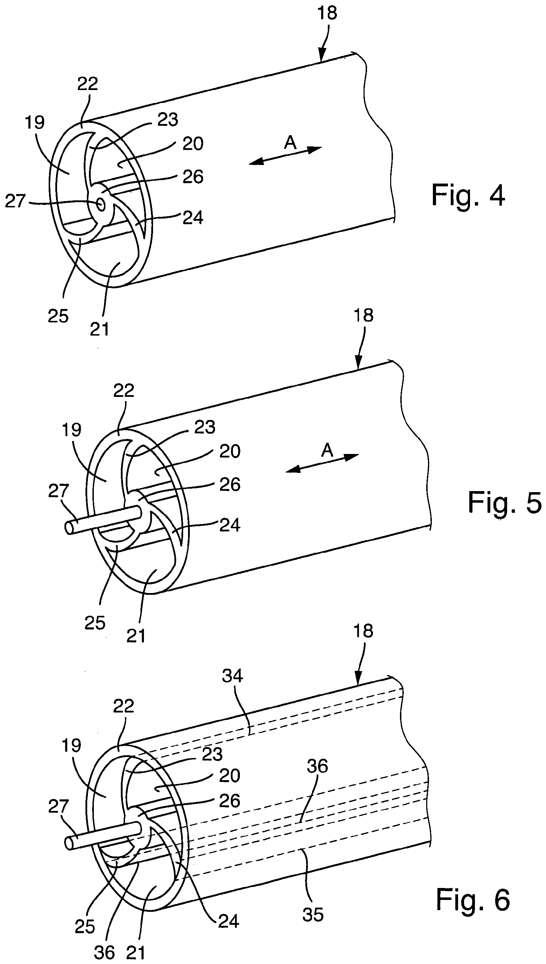

Der Schlauchköper 18 umschließt, wie Figur 3 erkennen lässt, mindestens ein, hier drei Lumina 19, 20, 21, die nach außen hin von einem Mantelbereich 22 und untereinander durch Wandbereiche 23, 24, 25 getrennt sind. Die Wandbereiche 23, 24, 25 erstrecken sich von dem Mantelbereich 22 zu einem Mittelbereich 26, mit dem sie nahtlos einstückig verbunden sind. Dieser Aufbau des Schlauchkörpers 18 ist insbesondere aus Figur 4 erkennbar. Der Schlauchkörper 18 bildet somit einen einteiligen, aus einheitlichem Material bestehenden nahtlosen Körper. Der Mittelbereich 26 kann einen im Wesentlichen runden oder anderweitigen (z.B. polygonalen) Querschnitt aufweisen und zentral zu dem ansonsten hohlzylindrischen Mantelbereich 22 angeordnet sein. Mittig in dem Mittelbereich 26 kann ein elektrischer Leiter 27 angeordnet sein, der sich über die gesamte Länge des Schlauchkörpers 18 durch den Mittelbereich 26 erstreckt. Der Leiter 27 kann ein blanker Leiter oder ein durch Lack oder einen Kunststoffmantel isolierter Leiter 27 sein. Die Wandbereiche 23, 24, 25 können, wie es bevorzugt wird, zur Radialrichtung geneigt sein. Die Wandbereiche 23, 24, 25 können flach oder gewölbt ausgebildet sein. Die Radialrichtung erstreckt sich radial von dem Leiter 27 weg, der seinerseits axial verläuft. In Figur 4 ist die Axialrichtung A durch einen Pfeil angedeutet. Die Radialrichtung ist jede dazu rechtwinklig stehende Richtung.

Das Endstück 17 weist einen Hohlkörper 28 auf, an dem die Öffnung 16 zum Beispiel als distale Endöffnung ausgebildet ist, wie es Figur 2 und 3 veranschaulichen. Die Öffnung 16 des ansonsten zentralen Durchgangskanals 29 kann jedoch auch seitlich an dem Körper 28 angebracht sein. Außerdem kann der Körper 28 mehrere solcher mit dem Durchgangskanal 29 verbundener Öffnungen 16 beispielsweise an seinem Umfang verteilt aufweisen. Das Distale Ende des Körpers 28 kann dann geschlossen sein.

Vorzugsweise ist der Körper 28 rotationssymmetrisch ausgebildet und umschließt einen Durchgangskanal 29, der an der Öffnung 16 endet. Der Durchgangskanal 29 erstreckt sich durch den Körper 28 und einem Rohrschaft 30, der sich an den Körper 28 anschließt und vorzugsweise einstückiger Bestandteil desselben ist. Der Rohrschaft 30 ist vorzugsweise hohlzylindrisch ausgebildet, wobei an seiner Außenumfangsfläche Verankerungsmittel 31, wie beispielsweise im Profil sägezahnartige Ringsicken 32, 33, einzelne Zähne oder Gewinde aufweisen kann. Der Außendurchmesser des Rohrschafts 30 ist vorzugsweise zumindest etwas größer als der Innendurchmesser des zylindrischen Mantelbereichs 22. An dem distalen Ende des Rohrschafts 30 ist im Übergang zu dem Körper 28 eine Ringschulter 34 ausgebildet, an der die distale Stirnfläche des Mantelbereichs 22 ihre Anlage finden kann.

Zur Verbindung des Endstücks 17 mit dem Schlauchkörper 18 wird wie folgt vorgegangen:

In einem ersten Schritt wird der Schlauchkörper 18 wie in Figur 4 veranschaulicht bereitgestellt. Der Schlauchkörper 18 kann als Endlosmaterial durch Extrusion gefertigt und zunächst mit einem geeigneten Schneidwerkzeug, wie einer Schere oder dergleichen, auf die gewünschte Länge zugeschnitten sein. An sein proximales Ende wird dann eine Zuleitung oder ein geeigneter Stecker angeschlossen, wobei der Raumbedarf desselben eine untergeordnete Rolle spielt. Besonderes Augenmerk liegt jedoch auf dem distalen Ende und dem Anschluss des Endstücks 17. Dazu wird, zumindest optional, von dem distalen Ende des Schlauchkörpers 18 mit einem geeigneten Schneidwerkzeug ein Teil abgetrennt, sodass der Leiter 27 frei liegt. Dies erleichtert später die Anbringung der Elektrode, wobei auf diesen Schritt auch verzichtet werden kann.

Der nächste im Zusammenhang mit der Anbringung des Endstücks 17 tatsächlich wichtige Schritt ist die Durchtrennung der Verbindung zwischen dem Mantelbereich 22 und den Wandbereichen 23, 24, 25. Dazu werden, wie Figur 6 andeutet, entsprechende Schnitte entlang der Innenumfangsfläche des Mantelbereichs 22 ausgebildet, welche Schnitte die Wandbereiche 23, 24, 25 im jeweiligen Übergang zu dem Mantelbereich 22 durchtrennen. Die Schnitte 34 bis 36 sind in Figur 6 durch gestrichelte Linien angedeutet. Sie erstrecken sich über eine axiale Distanz in Proximalrichtung in den Schlauchkörper 18 hinein welche Distanz vorzugsweise größer ist als die axiale Länge des Rohrschafts 30 (Figur 3) .

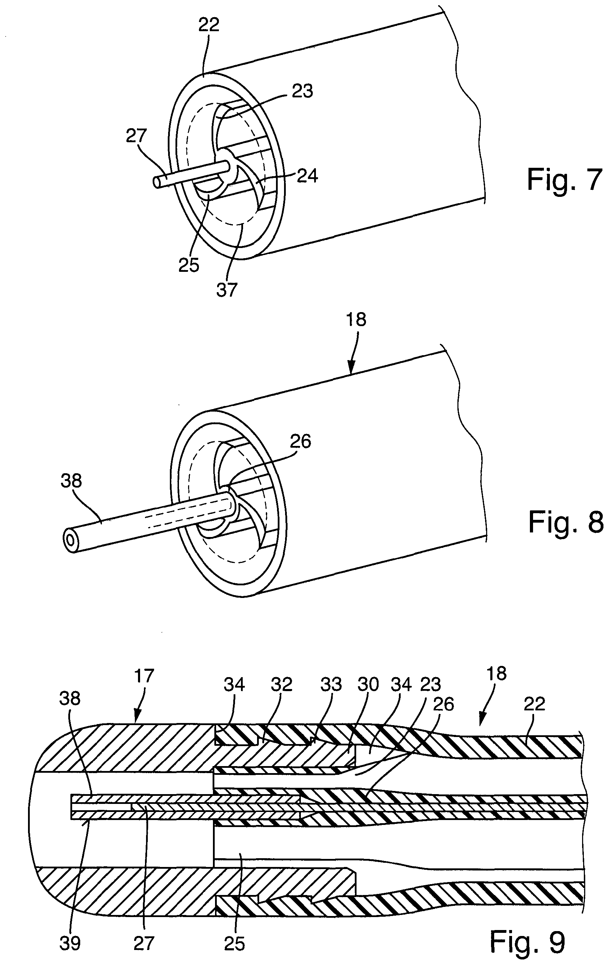

Zur Montage des Endstücks 17 an dem Schlauchkörper 18 wird der Rohrschaft 30 nun so in die Schnitte 34 bis 36 eingeschoben, dass er sich zwischen die Wandbereiche 23, 24, 25 und dem Mantelbereich 22 schiebt. Dabei werden die Wandbereiche 23, 24, 25, wie Figur 7 zeigt, radial nach innen verformt. Die Innenkontur des Rohrschafts 30 ist in Figur 7 durch eine gestrichelte Litze 37 markiert. Die schräg zur radialen Richtung orientierten Wände 23 bis 25 weichen radial nach innen aus und sind soweit etwas gebogen.

Das freigelegte Ende des Leiters 27 kann selbst als Elektrode dienen. Es kann jedoch zusätzlich oder alternativ ein Elektrodenkörper 38 auf das freigelegte Ende des Leiters 27 aufgeschoben und in den Mittelbereich 26 des Schlauchkörpers 18 eingeschoben sein, wie es Figur 8 und insbesondere Figur 9 zeigt. Figur 9 lässt insbesondere die Schlitze 34, 36 erkennen, die der Rohrschaft 30 unter Deformation der Wandbereiche 23, 25, (sowie des in Figur 9 weggeschnitten Wandbereichs 24) verursacht hat. Figur 9 lässt auch den Elektrodenkörper 38 erkennen, der als Röhrchen ausgebildet ist und innen den Leiter 27 aufnimmt, während er sich in den Mittelbereich 26 unter Aufweitung desselben erstreckt. Dies ist unabhängig davon, ob der Leiter 27 zunächst gemäß Figur 5 freigelegt worden ist oder ob die Schnitte 34, 35, 36 zur Montage des Endstücks, d.h. zur Aufnahme des Rohrschafts 30 direkt an den Schlauchkörper 18 nach Figur 4 vorgenommen worden ist.

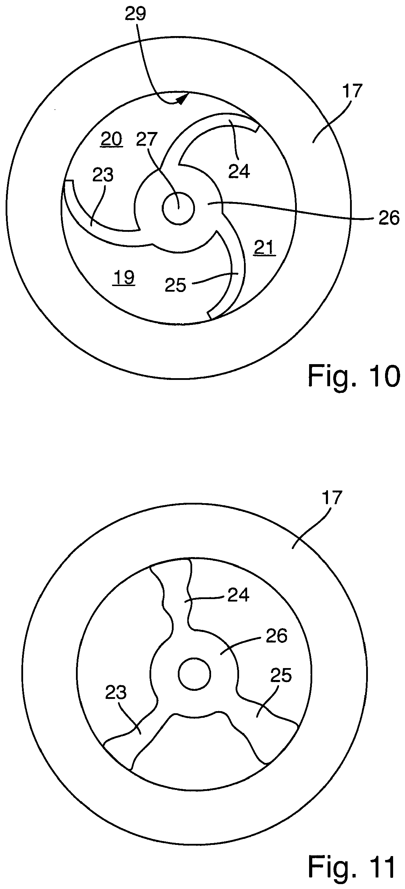

Figur 10 veranschaulicht die erfindungsgemäße Sonde nochmals mit einem Blick in den Durchgangskanal 29. Wie ersichtlich sind die etwa tangential von dem Mittelbereich 26 abgehenden radial außen freigeschnittenen Wandbereiche 23, 24, 25 von dem Endstück 17 nach innen verdrängt, sodass sie an der Wandung des Durchgangskanals 29 anliegen. Die Lumen 19, 20, 21 sind von dem Rohrschaft und den Wandbereichen 23, 24, 25 etwas eingeengt, was die Strömungsgeschwindigkeit des von dem Lumen 19, 20, 21 herangeführten Gases im Bereich des Endstücks 17 etwas erhöht.

Die Wandbereiche 23, 24, 25 müssen nicht zwingend schräg zur Radialrichtung angeordnet sein und somit tangential von dem Mittelbereich 26 abgehen. Sie können auch anderweitig angeordnet und beispielsweise gemäß Figur 11 radial orientiert sein. Das Einschieben des Rohrschafts den Endstücks 17 in die Schlitze zwischen den Wandbereichen 23, 24, 25 und dem Mantelbereich 22 kann dabei zu einer gewissen Stauchung der Wandbereiche 23 bis 25 führen, was in Figur 11 symbolisch überhöht veranschaulicht ist. Auf die Details der Funktion der Sonde 12 hat dies keinen Einfluss.

Im Betrieb speist das Gerät 14 die Lumina 19, 20, 21 der Sonde 12 mit einem geeigneten Gas, beispielsweise einem Inertgas, wie z.B. Argon. Dieses strömt dann durch die Lumen 19, 20, 21 von dem proximalen Ende 13 der Sonde 12 zu dem distalen Ende 15 und tritt dort aus der Öffnung 16 aus. Über das Gerät 14 wird der Leiter 27 und mit diesem der Elektrodenkörper 38 mit einer hohen Spannung gegenüber einer Neutralelektrode beaufschlagt, die an dem Patienten angebracht ist. Von dem Elektrodenkörper 38 geht somit eine Gasentladung aus, die den Gasstrom ionisiert sodass aus der Öffnung 16 ein Plasmastrahl austritt, über den der von dem Generator 14 gelieferte Strom zu dem Patienten und über die nicht weiter veranschaulichte Neutralelektrode zu dem Generator 14 zurückfließt. Insbesondere wenn der Elektrodenkörper 38 mit einem niedrigschmelzenden Metall 39, insbesondere Silber, beschichtet ist, konzentriert sich der Entladungsfußpunkt der Gasentladung auf das distale Ende des Elektrodenkörpers 38. Der Wärmeeintrag in den Mittelbereich 26 des Schlauchkörpers 18 kann dabei gering gehalten werden, insbesondere, wenn der Elektrodenkörper 38 aus einem thermisch schlecht leitenden Material, zum Beispiel Edelstahl besteht. Der Rohrschaft 30 verengt den freien Strömungsquerschnitt der Lumina 19, 20, 21. Der Elektrodenkörper 38 weitet den Mittelbereich 26 auf, wodurch die Lumen 19, 20, 21 weiter verengt sind. Dadurch wird eine Düsenwirkung im Beriech des Rohrschafts erzeugt, die zu einer guten Kühlung des Mittelbereichs beiträgt. Dadurch kann der Kunststoff 26 des Mittelbereichs den Elektrodenkörper 38 direkt berühren und halten ohne dadurch geschädigt zu werden.

Die Erfindung schafft ein Konzept zur zuverlässigen und einfachen Verbindung eines hitzefesten Endstücks 17 an einem Schlauchkörper 18, insbesondere einem mehrlumigen Schlauchkörper 18. Die erfindungsgemäße Verbindungstechnik ist einfach und verlässlich und führt zu langlebigen Sonden hoher Qualität.

### Bezugszeichen:

- 12: Sonde
- 13: proximales Ende der Sonde
- 14: Gerät
- 15: distales Ende
- 16: Öffnung
- 17: Endstück
- 18: Schlauchkörper
- 19 - 21: Lumina
- 22: Mantelbereich
- 23 - 25: Wandbereiche
- 26: Mittelbereich
- 27: Leiter
- A: Axialrichtung
- 28: Körper
- 29: Durchgangskanal
- 30: Rohrschaft
- 31: Verankerungsmittel
- 32 - 33: Rippen (Ringsicken)
- 34 - 36: Schnitte
- 37: Ellipse
- 38: Elektrodenkörper
- 39: Beschichtung

## Patentansprüche

1. Plasmasonde (12), insbesondere zur endoskopischen Behandlung menschlicher oder tierischer Patienten,
mit einem Schlauchkörper (18), der einen Mantelbereich (22), welcher mindestens ein Lumen (19, 20, 21) begrenzend ausgebildet ist, und einen Mittelbereich (26) aufweist, der mit dem Mantelbereich (22) über mindestens einen Wandbereich (23) verbunden ist,
mit einem Endstück (17), das an einem distalen Ende (15) des Schlauchkörpers (18) angeordnet ist und einen Durchgangskanal (29) sowie einen Rohrschaft (30) aufweist, der sich in den Schlauchkörper (18) hinein erstreckend angeordnet ist,
wobei zwischen dem Mantelbereich (22) und dem Wandbereich (23, 24, 25) jeweils ein Schlitz (34, 35, 36) ausgebildet ist, der sich von dem distalen Ende (15) des Schlauchkörpers (18) in proximaler Richtung erstreckend ausgebildet ist,
wobei der Rohrschaft (30) in den Schlitz (34, 35, 36) ragend angeordnet ist.

2. Plasmasonde nach Anspruch 1, **dadurch gekennzeichnet, dass** der Schlauchkörper (18) aus einem flexiblen Kunststoff besteht.

3. Plasmasonde nach einem der vorstehenden Ansprüche, **dadurch gekennzeichnet, dass** der Mantelbereich (22), der Wandbereich (23) und der Mittelbereich (26) untereinander nahtlos einstückig verbunden sind.

4. Plasmasonde nach einem der vorstehenden Ansprüche, **dadurch gekennzeichnet, dass** der Schlauchkörper (18) über seine gesamte Länge einen konstanten Querschnitt aufweisend ausgebildet ist.

5. Plasmasonde nach einem der vorstehenden Ansprüche, **dadurch gekennzeichnet, dass** das Endstück (17) aus einem hitzefesten Kunststoff, aus einem Metall oder aus Keramik ausgebildet ist

6. Plasmasonde nach einem der vorstehenden Ansprüche, **dadurch gekennzeichnet, dass** der Schlitz (34, 35, 36) eine Länge aufweist, die größer ist als die Länge des Rohrschafts (30).

7. Plasmasonde nach einem der vorstehenden Ansprüche, **dadurch gekennzeichnet, dass** der Durchgangskanal (29) des Rohrschafts (30) eine Wandung aufweist, an der der Wandbereich (23) mit seinem dem Mantelbereich (22) zugewandten Ende anliegt.

8. Plasmasonde nach Anspruch 7, **dadurch gekennzeichnet, dass** der Mantelbereich (22) zumindest im Bereich des Rohrschafts (30) zylindrisch ausgebildet ist.

9. Plasmasonde nach einem der vorstehenden Ansprüche, **dadurch gekennzeichnet, dass** der Wandbereich (23) innerhalb des Rohrschafts (30) deformiert ist.

10. Plasmasonde nach einem der vorstehenden Ansprüche, **dadurch gekennzeichnet, dass** der Rohrschaft (30) an seiner radial äußeren Fläche mit einer Verankerungsstruktur (31) versehen ist.

11. Plasmasonde nach einem der vorstehenden Ansprüche, **dadurch gekennzeichnet, dass** in dem Mittelbereich (26) ein elektrischer Leiter (27) angeordnet ist.

12. Plasmasonde nach einem der vorstehenden Ansprüche, **dadurch gekennzeichnet, dass** in dem Mittelbereich (26) ein Elektrodenkörper (38) gehalten ist.

13. Plasmasonde nach Anspruch 12, **dadurch gekennzeichnet, dass** der Elektrodenkörper (38) rohrförmig ausgebildet ist und sich zwischen den Leiter (27) und den Mittelbereich (26) erstreckt.

14. Plasmasonde nach einem der vorstehenden Ansprüche, **dadurch gekennzeichnet, dass** der Elektrodenkörper (38) aus einem hitzebeständigen, elektrisch leitfähigen Material besteht und zumindest an seiner Oberfläche mit einer elektrisch leitfähigen Beschichtung (39) versehen ist, deren Schmelztemperatur niedriger ist, als die des Materials des übrigen Elektrodenkörpers (38).

15. Verfahren zur Herstellung einer Plasmasonde, insbesondere zur endoskopischen Behandlung menschlicher oder tierischer Patienten, mit folgenden Schritten:
Bereitstellung eines Schlauchkörpers (18), der einen Mantelbereich (22), welcher mindestens ein Lumen (19, 20, 21) begrenzend ausgebildet ist, und einen Mittelbereich (26) aufweist, der mit dem Mantelbereich (22) über mindestens einen Wandbereich (23) verbunden ist,
Bereitstellung eines Endstücks (17), das an einem distalen Ende (15) des Schlauchkörpers (17) anzuordnen ist und einen Durchgangskanal (29) sowie einen Rohrschaft (30) aufweist,
Ausbilden eines Schlitzes (34) zwischen dem Mantelbereich (22) und dem Wandbereich (23) ausgehend von dem distalen Ende (15) des Schlauchkörpers (18) in proximaler Richtung,
Einschieben des Rohrschafts (30) in den Schlitz (34) unter Verdrängung des Wandbereichs (23) aus dem Schlitz (34).
